Europäisches Patentamt

(19) European Patent Office

Office européen des brevets

(11) Publication number: **0 117 470 B1**

(12) **EUROPEAN PATENT SPECIFICATION**

(45) Date of publication of patent specification: **09.09.87**

(21) Application number: **84101404.6**

(22) Date of filing: **10.02.84**

(51) Int. Cl.⁴: **A 61 K 45/02, C 07 K 15/26, B 01 D 15/08**

(54) Process for producing interferon (IFN-gamma) subtypes 26K and 21K.

(30) Priority: **13.02.83 IL 67896**

(43) Date of publication of application:
**05.09.84 Bulletin 84/36**

(45) Publication of the grant of the patent:
**09.09.87 Bulletin 87/37**

(84) Designated Contracting States:
**BE CH DE FR GB IT LI NL SE**

(56) References cited:
**WO-A-83/00444**
**DE-A-2 947 134**
**GB-A-2 061 285**

**YIP et al., Science 215, 411-413**

(73) Proprietor: **YEDA RESEARCH AND DEVELOPMENT COMPANY, LIMITED**
**P.O. Box 95**
**Rehovot 76100 (IL)**

(72) Inventor: **Rubinstein, Menachem**
**16, Hatomer Street**
**Givat Shmuel (IL)**
Inventor: **Friedlander, Jossef**
**19 Herzl Street**
**Bat Yam (IL)**
Inventor: **Fischer, Dina**
**8, Ahronson Street**
**Ramat Gan (IL)**

(74) Representative: **Patentanwälte Grünecker, Kinkeldey, Stockmair & Partner**
**Maximilianstrasse 58**
**D-8000 München 22 (DE)**

Courier Press, Leamington Spa, England.

## Description

Among the various types of human interferons, interferon gamma is the least characterized. This interferon is produced in lymphocytes upon stimulation with mitogens or specific antigens and it differs significantly in its structure and properties from the virus-induced alpha and beta interferons.

Recently, Yip et al., in Proc. Nat. Acad. Sci. USA 78, 1601 (1981) have reported a method of human interferon gamma production based on the stimulation of lymphocytes by a combination of the phorbol ester 12-0-tetradecanoyl-phorbol-13 acetate (TPA) and phytohemagglutinin (PHA). Later Yip et al., were able, as reported in Science 215, 411 (1982) and Proc. Nat. Acad. Sci. 79, 1820 (1982) to purify by a preparative Na Dod So₄ polyacrylamide gel electrophoresis SDS-PAGE, two subtypes of interferon gamma having apparent molecular weights of 21K and 26K. A third minor component having an apparent molecular weight of about 43K was also detected. The isolation of these species by SDS-PAGE is highly undesirable because SDS sharply reduces the activity of the interferon (to 10—20% of the original activity) and also remains present as a toxic contaminant. Partially purified interferon gamma was obtained according to Yip (PNAS 79, 1820 (1982)) by (1) CPG chromatography; (2) Con-A Sepharose chromatography and (3) DEAE-Sephacel chromatography. This preparation was then subjected to SDS-PAGE.

In another study, disclosed in Nature 295, 503 (1982) by Gray et al. the cloning and expression of human interferon gamma complementary DNA was reported. The nucleotide sequence indicated that this interferon gamma was composed of a single polypeptide with 146 amino acids and a calculated molecular mass of 17,000 daltons. Furthermore, analysis of a gene library did not reveal any other structurally related DNA sequences. It was suggested that there is only one polypeptide sequence related to interferon gamma and that native interferon gamma may be partly dimeric. Pestka and Rubinstein have previously described in U.S. Pat. 4,289,690 the purification to homogeneity of alpha interferon and the separation of eight distinct subtypes by reversed phase high performance liquid chromatography (HPLC), employing organic solvents and/or low pH. This method is not suitable for purification of interferon gamma because interferon gamma is labile to organic solvents and low pH. See also Rubinstein, et al., PNAS 76, 640 (1979); Rubinstein, Anal. Biochem., 97, 1 (1979); Rubinstein, et al., Arch. Biochem. Biophys. 210, 307 (1981).

Edy, in U.S. Patent No. 4,168,261 discloses controlled pore glass chromatography of interferon, with acid elution. The example given were of purification of human fibroblast interferon and mouse L-929 interferon. In both cases, it was admitted that the eluted interferon still contained contaminating protein. No examples were given of purification of interferon gamma which is acid-labile.

Braude in U.S. Patent Nos. 4,382,027, and 4,440,675 teaches the purification of crude immune interferon to a "near homogeneous preparation" by the combination of: (a) CPG chromatography, (b) carbohydrate affinity chromatography, (c) dye affinity chromatography (d) cation exchange chromatography and (e) gel filtration. There is no recognition of the existence of distinct subtypes of IFN gamma and the preparation is admittedly not homogeneous.

Braude in European Patent Applications No. 63,482, states that when analyzed by SDS-PAGE, the eluate from his multistep purification of IFN gamma demonstrated four bands (20K, 25K, 47K and 63K). Braude urged that all four bands represented subspecies or aggregates of IFN gamma, and therefore asserted that he had purified IFN gamma to homogeneity. According to Braude, the 25K, 47K and 63K bands all had essentially the same amino acid content and the 25K and 47K species were shown to possess IFN gamma activity.

Braude has failed to teach a method of preparing subspecies in a homogeneous, biologically active form. His eluate is, at best, only partially purified, since by his own admission it contains two distinct interferon gamma proteins and two other protein bands. Braude can separate these proteins by SDS-PAGE, but then the comments made above with respect to Yip et al. (PNAS 79, 1820 (1982)) apply. Moreover, at least the 63K protein might have been a contaminating non-interferon protein.

Braude's EP Appl. No. 139,878 claims priority from the same U.S. application as EP Appl. 63,482 and appears to be substantially identical to the latter.

While several of the above papers contain claims to have purified human immune interferons to homogenity, an almost complete loss (80—90%) of biological activity was admitted. Furthermore, none of the properties of the allegedly pure compounds were described.

The use of high performance liquid chromatography for purification of proteins is generally known in the art. The recent availability of ion exchange HPLC columns suitable for fractionation of protein has allowed high resolution chromatography of interferon gamma preparations.

It has been known in the art to employ chromatography on controlled-pore glass as a procedural step in the purification of interferon gamma (see Yip et al. PNAS 79, 1820 (1982)). Ultrafiltration was broadly used in the past for concentration and desalting of proteins. In some cases, ultrafiltration could be used to remove peptides and proteins having a molecular weight lower than the cut-off value of the ultrafiltration membrane. Mono-S cation exchange HPLC columns were designed and marketed by Pharmacia Fine Chemicals specifically for the fractionation of proteins. However, the prior art did not utilize or suggest the use of these HPLC columns in conjunction with controlled pore glass chromatography and ultrafiltration for the fractionation of interferon gamma.

An object of the invention is a human interferon gamma species, of subtype 21K in essentially

homogeneous and biologically active form having an approximate molecular mass of 21,000 and a specific activity of at least about $7 \times 10^6$ units/mg.

Another object of the invention is a human interferon gamma species, of subtype 26K in essentially homogeneous and biologically form having an approximate molecular mass of 26,000 and a specific activity of at least about $7 \times 10^6$ units/mg.

The invention also relates to a process for producing the above human interferon gamma species, which comprises in combination:

(a) Mixing an aqueous solution of (IFN-γ) in an impure state with a controlled-pore glass material so as to adsorb said interferon onto said adsorbent and thereafter eluting said interferon from said adsorbent and obtaining said interferon in selected fractions of said eluate in a state of enhanced purity;

(b) concentrating the selected said fractions obtained in step (a) by ultrafiltration on a membrane and obtaining said interferon in the retained fraction of said membrane in a state of higher concentration and an enhanced purity;

(c) passing said fraction obtained in step (b) through a cation exchange column where said column is equilibrated with an appropriate buffer and thereafter eluting said interferon from said column with a gradient of increasing salt concentration or alternatively increasing pH and under moderate pressure and obtaining subtypes 26K any 21K of IFN-γ as several distinct peaks in separate fractions of said eluate, in the state of homogeneous and biologically active proteins. In some cases, ultrafiltration could be used to remove peptides and proteins having a molecular weight lower than the cut-off value of the ultrafiltration membrane. Mono-S® cation exchange HPLC columns were designed and marketed by Pharmacia Fine Chemicals specifically for the fractionation of proteins.

Since an international standard of IFN-γ does not exist yet, the biological activity given here and elsewhere in this invention is calibrated against interferon-α reference standard G-023-901-527 supplied by the National Institutes of Health (Bethesda, Maryland, U.S.A.) and utilizes human WISH cells (American Type Culture Collection Cat. No. CCL-23) and vesicular stomatitis virus in an assay based on the inhibition of the viral cytophatic effect. It is generally known in the art that the use of other cell lines, other viruses, and other standards may give a very wide range of specific activities. An additional variable is the protein content which may be determined by various procedures. The protein content in the present invention was based on an amino acid analysis which is widely accepted as an absolute method.

The use of controlled-pore glass chromatography on crude IFN-γ preparations results in about a 4-fold purification. Thus, IFN-γ produced by this step has a specific activity of about $2 \times 10^5$ units/mg. For controlled-pore glass (CPG) procedure the following protocol can be employed.

CPG beads were added to culture supernatants containing IFN-γ (3,000—16,000 units/ml), 8—$20 \times 10^4$ units/mg) at a ratio of 1:100 (vol/vol) in a polypropylene centrifuge bottle. The mixture was stirred for 3 h at 4°C, the beads were allowed to settle, the supernatant was aspirated and the beads were packed into a siliconized glass column. The column was first washed with several column volumes of phosphate-buffered saline, and IFN-γ was eluted by 0.5M tetramethylammonium chloride pH 7.

The resulting purified interferon obtained by elution from CPG with tetramethylammonium chloride· exhibits a specific activity of about $2 \times 10^5$ units/mg with a recovery of about 50—100%. Fractions containing IFN-γ activity from the CPG-chromatography were combined and salt was removed by ultrafiltration on a PM-10 membrane (Amicon). The retained fraction was washed with several volumes of 2 mM sodium phosphate (pH 7.4) and concentrated to about 0.5% of the original culture volume. Insoluble proteins were removed by centrifugation and the clear supernatant was stored at 4°C until used. The resulting interferon concentrate obtained by ultrafiltration exhibits a specific activity of $9 \times 10^5$ units/mg with a recovery of about 60—100%.

In order to achieve further purification of the interferon concentrate produced by ultrafiltration, the interferon is processed through one or two cation exchange high pressure liquid chromatography (HPLC) steps with high resolution. The liquid chromatography step utilizes columns containing a monodisperse organic polymeric matrix to which sulfoalkyl groups are bonded. These columns, which can be used sequentially and under varying conditions of pH gradient and ionic strength gradients and in the presence of various stabilizers such as ethylene-glycol can purify human immune interferon to homogeneity as determined by obtaining a single band on sodium-dodecyl sulfate (SDS) polyacrylamide gel electrophoresis, a constant specific activity and a single peak on HPLC with activity and protein levels superimposable.

The organic matrix cation-exchange HPLC columns used in the practice of the invention are articles of commerce. A suitable column is the Mono-S® produced and marketed by Pharmacia Fine Chemicals, Uppsala, Sweden. A high pressure liquid chromatography system; for utilizing the aforesaid column, is available from a large number of manufacturers such as Pharmacia, Varian, Beckman or Waters or Perkin Elmers.

A preferred buffer system for eluting interferon-γ from the HPLC column in the present invention is 10 mM sodium phosphate pH 7.0, 20% vol/vol ethylene glycol. The procedure is carried out under a moderate pressure from 0.7 to 70 bar. The flow rate used for a standard $5 \times 50$ mm column is from 0.1 to 1.0 ml/min and the procedure can be performed at a range of temperatures from −10°C to +30°C. Interferon-γ (from 0.1 mg to 10 mg protein) is adsorbed to the column and is then subsequently eluted in selective fashion using a gradient of increasing salt concentration. Suitable salts for this purpose include NaCl, KCl, $MgCl_2$,

MgSO$_4$ and tetramethylammonium chloride. Separation can be obtained in any pH value of a range varying from pH 5 to pH 8.5. In an alternative method the salt concentration remains constant and the pH is varied in an increasing gradient from pH 5 to pH 10. Further purifications can be obtained by performing two or more consecutive fractionations on the same column at either the same conditions or at different conditions as outlined above.

Fractionation of the eluate is accomplished by utilizing fraction collectors in a manner known *per se* with concomitant monitoring of the protein content during fractionation by a protein monitor such as an ultraviolet detector operating at any wavelength in the range of 210—280 nm and preferably at either 210 or 280 nm.

Due to the possible loss of IFN-γ by adsorption to silica and to glass, all tubings, containers and test-tubes under the present invention were made of either Teflon®, polypropylene or polyethylene.

The highly purified preparations were most stable when stored at +4°C in polypropylene test tubes and in the presence of 20% vol/vol ethylene glycol. Significant loss of biological activity was noticed in repeated freezing-thawing cycles.

Four major peaks of biological activity eluting at NaCl concentrations of 0.15, 0.20, 0.25 and 0.26 molar (at pH 7.0) were obtained with an overall recovery of 20—40%. Since the separation was performed under non denaturing conditions each peak represented a discrete subtype of HuIFN-γ. Analysis by NaDodSO$_4$-polyacrylamide gel electrophoresis either in then presence or in the absence of β-mercaptoethanol revealed that the peaks eluting at 0.25M and 0.26M NaCl contained single protein bands with apparent molecular masses of 26,000 (26K), and 21,000 (21K) respectively, while the peaks at 0.15M and 0.20M were less pure. The specific activity of both subtypes 26K and 21K was $7 \times 10^6$ units/mg. Analysis by gel filtration on a Ultrogel® AcA54 column of either crude or any of the purified IFN-γ subtypes under non denaturing conditions gave a peak of biological activity corresponding to a molecular mass of 45,000 daltons. Amino acid analysis of subtypes 26K and 21K showed a general similarity to the theoretical values calculated from the sequence of the cDNA clone as described by Gray *et al.* (4). However, significant differences in the levels of proline, glycine and panylalanine were observed. Subtypes 26K and 21K gave almost identical amino acid compositions and peptide maps.

Interferon-γ have exhibited antiviral activity, anti cellular activity, ability to augment natural and antibody dependent killer cell activity, monocyte and macrophage activation properties and ability to induce certain HLA surface marker antigens. These activities have been obtained with relatively crude preparations containing a large number of biologically active lymphokines. The purified homogeneous human immune interferons of the present invention can be utilized in order to identify those activities belonging to IFN-γ proper. Preparations containing the purified IFN-γ can be used for clinical studies to estimate their usefulness in a variety of viral and neoplastic diseases.

The process and product aspects of this invention are further illustrated by reference to the following examples:

Example 1:
Production of human IFN-γ.

Mononuclear cells were isolated from buffy coats by centrifugation (400×g, 30 min) on a Ficoll-Hypaque gradient. IFN-γ was induced in cultures of mononuclear cells ($5 \times 10^6$/ml) in serum-free RPM1-1640 medium by addition of 12-0-tetradecanoyl phorbol-13-acetate (5 ng/ml) and purified phytohemagglutinin (5 µg/ml). After 24 hrs of incubation at 37°C and a 5% CO$_2$ atmosphere, cells were removed by centrifugation, culture supernatants were collected and stored at +4°C.

Example 2: Process according to the invention.
Steps (a) and (b):
Chromatography on controlled-pore glass and ultrafiltration.

CPG beads were added to culture supernatants containing IFN-γ (3,000—16,000 units/ml), $8—20 \times 10^4$ units/mg) at a ratio of 1:100 (vol/vol) in a polypropylene centrifuge bottle. The mixture was stirred for 3 h at 4°C, the beads were allowed to settle, the supernatant was aspirated and the beads were packed into a siliconized glass column. The column was first washed with several column volumes of phosphate-buffered saline, and IFN-γ was eluted by 0.5M tetramethylammonium chloride pH 7. Fractions containing IFN-γ activity from the CPG-chromatography were combined and salt was removed bq ultrafiltration on a PM-10 membrane (Amicon). The retained fraction was washed with several volumes of 2 mM sodium phosphate (pH 7.4) and concentrated to about 0.5% of the original culture volume. Insoluble proteins were removed by centrifugation and the clear supernatant was stored at +4°C until used.

Step (c):
High performance liquid chromatography (HPLC).

HPLC was performed on an Altex Model 330 liquid chromatograph (Beckman Instruments). A Mono-S HPLC cation-exchange column (5×50 mm) was equilibrated with 10 mM sodium phosphate pH 7.0—20% (vol/vol) ethylene glycol (Buffer A). IFN-γ preparation from the previous step was loaded at a flow rate of 0.5 ml/min. The column was then washed with Buffer A for 30 min followed by a 60 min linear gradient of NaCl (0—400 mM) in Buffer A. Each fraction (1 ml) was then assayed for IFN-γ activity and for protein content using bovine serum albumin as a protein standard. High resolution was achieved only when ethylene glycol was included in the elution buffers. Several peaks of biological activity eluting at NaCl concentrations of 0.15M, 0.20M, 0.25M and 0.26M were consistently obtained. Usually the peak at 0.25M was the most predominant. The peaks of biological activity at 0.25M and 0.26M were associated with two distinct protein peaks and their specific activity in various preparations was $7 \times 10^6$ units/mg. The peaks of biological activity at lower NaCl concentrations (0.15M and 0.20M) had lower specific activities and could not be associated with discrete protein peaks. The total recovery of biological activity was 20—40% with losses mainly at the last step.

Example 3: Analysis.
NaDodSO$_4$-polyacrylamide gel electrophoresis and amino acid analysis.
Protein samples were first dialyzed against 2 mM sodium phosphate pH 7.4, dried in a Speedvac Concentrator (Savant), dissolved in a freshly prepared sample buffer with 2% β-mercaptoethanol and heated for 5 min at 100°C. A slab gel of 15% polyacrylamide was used. After electrophoresis, protein bands were visualized either with Coomassie blue or by silver stain. The fraction eluting at 0.26M salt gave a single protein band with an apparent molecular mass of 21,000 daltons (subtype 21K). The fraction eluting at 0.25M salt gave a major protein band with an apparent molecular mass of 26,000 (subtype 26K), but some minor bands of very low molecular masses were occasionally seen. Rechromatography of this fraction on the same HPLC column had eliminated these contaminants. The peaks of biological activity eluting at lower salt concentrations (0.15—0.20 M) exhibited several major protein bands including a band corresponding to M 26,000. The apparent molecular masses of peaks 21K and 26K were not affected by treatment with β-mercaptoethanol prior to gel electrophoresis.

Amino acid analysis.
Acetone (4—5 volumes) was added to samples containing IFN-γ (10—30 µg) from the Mono-S® column. The mixtures were left 3—16 h at −20°C, spun (13,000×g, 5 min) and the precipitate was dried in a vacuum. Amino acid analysis was performed on a Durrum® 500 analyser after hydrolysis (6N HCl, 110°C, 24 h). No correction for Serine or Threonine was made. Amino acid analysis of both subtypes 21K and 26K gave values quite similar to those calculated from the sequence of the gene. The high content of glycine was probably due to contaminants and to destruction of other amino acids during hydrolysis.

Amino acid compositions of IFN-γ

| Amino acid | Subtype 21K | Subtype 26K |
|---|---|---|
| Asx | 14.6±1.3 | 16.5±0.4 |
| Thr | 7.9±0.4 | 7.4±0.6 |
| Ser | 9.1±1.4 | 10.8±1.0 |
| Glx | 15.6±0.7 | 16.4±1.4 |
| Pro | 9.7±1.4 | 8.0±0.4 |
| Gly | 14.4±0.3 | 14.3±1.9 |
| Ala | 12.0±1.5 | 11.3±0.7 |
| Cys | 0.6±0.5 | 0.6±0.4 |
| Val | 7.4±0.7 | 8.9±0.9 |
| Met | 2.3±1.0 | 0.8±0.3 |
| Ile | 5.0±0.3 | 6.0±0.3 |
| Leu | 9.2±0.3 | 12.9±0.1 |
| Tyr | 2.9±0.4 | 2.1±0.1 |
| Phe | 4.9±1.0 | 5.2±0.8 |
| His | 3.5±0.7 | 2.2±0.3 |
| Lys | 20.3±0.4 | 13.6±3.1 |
| Arg | 5.7±0.4 | 8.4±1.0 |

Example 4:
Parental dosage form with homogeneous human immune interferon subtype 26K.
A total of 10 mg of homogeneous human immune interferon subtype 26K having a specific activity of $7 \times 10^6$ units/mg is dissolved in 35 ml of normal serum albumin (human) USP. The solution is dialyzed against phosphate buffer saline with several changes to eliminate low molecular weight contaminants and then passed through a bacteriological filter. The filtered solution is aseptically subdivided into 140 vials. Each vial contains $5 \times 10^5$ units of the pure interferon suitable for parental administration. The vials are preferably stored in the cold (−20° to −70°C) prior to use.

Example 5:
Parental dosage form with homogeneous human immune interferon subtype 21K.
A total of 10 mg of homogeneous human immune interferon subtype 21K having a specific activity of

$7 \times 10^6$ units/mg is dissolved in 35 ml of normal serum albumin (human) USP. The solution is dialyzed against phosphate buffer saline with several changes to eliminate low molecular weight contaminants and then passed through a bacteriological filter. The filtered solution is aseptically subdivided into 140 vials. Each vial contains $5 \times 10^5$ units of the pure interferon suitable for parental administration. The vials are preferably stored in the cold ($-20°$C to $-70°$C) prior to use.

Example 6:
Parental dosage form with homogeneous human immune interferon subtype 26K and 21K.
A combination of 5 mg of homogeneous human immune interferon subtype 21K and 5 mg of homogenous human immune interferon subtype 26K, both having a specific activity of $7 \times 10^6$ units/mg is dissolved in 35 ml of normal serum albumin (human) USP. The solution is dialyzed against phosphate buffer saline with several changes to eliminate low molecular weight contaminants and then passed through a bacteriological filter. The filtered solution is aseptically subdivided into 140 vials. Each vial contains $5 \times 10^5$ units of the pure interferon suitable for parental administration. The vials are preferably stored in the cold ($-20°$C to $-70°$C prior to use.

**Claims**

1. A human interferon gamma species, of subtype 21K in essentially homogeneous and biologically active form having an approximate molecular mass of 21,000 and a specific activity of at least about $7 \times 10^6$ units/mg.

2. A human interferon gamma species, of subtype 26K in essentially homogeneous and biologically active form having an approximate molecular mass of 26,000 and a specific activity of at least about $7 \times 10^6$ units/mg.

3. The interferon species of claim 1, having essentially the following amino acid composition:

| Amino acid | Residues |
|---|---|
| Asx | $14.6 \pm 1.3$ |
| Thr | $7.0 \pm 0.4$ |
| Ser | $9.1 \pm 1.4$ |
| Glx | $15.6 \pm 0.7$ |
| Pro | $9.7 \pm 1.4$ |
| Gly | $14.4 \pm 0.3$ |
| Ala | $12.0 \pm 1.5$ |
| Cys | $0.6 \pm 0.5$ |
| Val | $7.4 \pm 0.7$ |
| Met | $2.3 \pm 1.0$ |
| Ile | $5.0 \pm 0.3$ |
| Leu | $9.2 \pm 0.3$ |
| Tyr | $2.9 \pm 0.4$ |
| Phe | $4.9 \pm 1.0$ |
| His | $3.5 \pm 0.7$ |
| Lys | $20.3 \pm 0.4$ |
| Arg | $5.7 \pm 0.4$ |

4. The interferon species of claim 2, having essentially the following amino acid composition:

| Amino acid | Residues |
|---|---|
| Asx | $16.5 \pm 0.4$ |
| Thr | $7.4 \pm 0.6$ |
| Ser | $10.8 \pm 1.0$ |
| Glx | $16.4 \pm 1.4$ |
| Pro | $8.0 \pm 0.4$ |
| Gly | $14.3 \pm 1.9$ |
| Ala | $11.3 \pm 0.7$ |
| Cys | $0.6 \pm 0.4$ |
| Val | $8.9 \pm 0.9$ |
| Met | $0.8 \pm 0.3$ |
| Ile | $6.0 \pm 0.3$ |
| Leu | $12.9 \pm 0.1$ |
| Tyr | $2.1 \pm 0.1$ |
| Phe | $5.2 \pm 0.8$ |
| His | $2.2 \pm 0.3$ |
| Lys | $13.6 \pm 3.1$ |
| Arg | $8.4 \pm 1.0$ |

5. The interferon species of claim 1, which elutes at 0.26M NaCl (pH 7) from a HPCL cation exchange 5×50 mm column at a flow rate of 0.1 to 1.0 ml/min, a temperature of −10°C to 30°C, and a pressure of 0.7 to 70 bar, after equilibration in 10 mM sodium phosphate (pH 7), 20% vol/vol ethylene glycol.

6. The interferon species of claim 2, which elutes at 0.25 M NaCl (pH 7) from a HPCL cation exchange 5×50 mm column at a flow rate of 0.1 to 1.0 ml/min, a temperature of −10°C to 30°C and a pressure of 0.7 to 70 bar, after equilibration in 10 mM sodium phosphate (pH 7) 20% vol/vol ethylene glycol.

7. A pharmaceutical preparation suitable for parenteral administration for treatment of viral and neoplastic disease states, comprising a minor effective amount of the interferon species of claim 1 and a major amount of a conventional pharmaceutical parenteral carrier material.

8. A pharmaceutical preparation suitable for parenteral administration for treatment of viral and neoplastic disease states, comprising a minor effective amount of the interferon species of claim 2 and a major amount of a conventional pharmaceutical parenteral carrier material.

9. A composition comprising a mixture of (a) a human gamma interferon species, of subtype 21K according to claim 1, in essentially homogeneous and biologically active form, and (b) a human gamma interferon species, of subtype 26K, according to claim 2, in essentially homogeneous and biologically active form.

10. A pharmaceutical preparation suitable for parenteral administration for treatment of viral and neoplastic disease states, comprising a minor effective amount of composition of claim 9 and a major amount of a conventional pharmaceutical parenteral carrier material.

11. A process for producing human immune interferon (IFN-γ) subtypes 21K according to claim 1 and 26K according to claim 2, as essentially homogeneous and biologically active proteins which comprises in combination:

a. Mixing an aqueous solution of (IFN-γ) in an impure state with a controlled-pore glass material so as to adsorb said interferon onto said adsorbent and thereafter eluting said interferon from said adsorbent and obtaining said interferon in selected fractions of said eluate in a state of enhanced purity;

b. concentrating the selected said fractions obtained in step (a) by ultrafiltration on a membrane and obtaining said interferon in the retained fraction of said membrane in a state of higher concentration and an enhanced purity;

c. passing said fraction obtained in step (b) through a cation exchange column where said column is equilibrated with an appropriate buffer and thereafter eluting said interferon from said column with a gradient of increasing salt concentration or alternatively an increasing pH and under moderate pressure and obtaining subtypes 21K and 26K of IFN-γ as several distinct peaks in separate fractions of said eluate, in the state of homogeneous and biologically active proteins.

12. The process of claim 11 in which step (c) is carried out under HPLC conditions.

13. The process of claim 11 in which the cation exchange column comprises an organic polymer bearing sulfoalkyl groups.

14. The process of claim 11 in which the interferon is eluted in step (c) by increasing salt concentration.

15. The process of claim 11 in which the interferon is eluted in step (c) by increasing pH.

16. The process of claim 11 in which the interferon is eluted in step (c) in the presence of ethylene glycol.

17. The process of one of the claims 11 to 16 wherein said column is a cation exchange column comprising an organic polymer bearing sulfoalkyl groups, said buffer is 10 mM sodium phosphate pH 7.0 containing 20% vol/vol ethylene glycol and said gradient is 60 min of linearly increased NaCl concentration from 0 to 400 mM.

18. The process of one of the claims 11 to 17 in which in at least one step (c) fraction, subtype 21K is eluted essentially free of subtype 26K, and in at least one other step (c) fraction, subtype 26K is eluted essentially free of subtype 21K, whereby subtypes 21K and 26K are coproduced in essentially homogeneous form.

19. The process of claim 11 in which the membrane of step (b) has a molecular weight cutoff of about 10,000 daltons.

**Patentansprüche**

1. Eine menschliche Interferon-gamma-Art des Subtyps 21K in im wesentlichen homogener und biologisch aktiver Form, die eine ungefähre Molekularmasse von 21,000 und eine spezifische Aktivität von mindestens $7 \times 10^6$ Einheiten/mg aufweist.

2. Eine menschliche Interferon-gamma-Art des Subtyps 26K in im wesentlichen homogener und biologisch aktiver Form, die eine ungefähre Molekularmasse von 26.000 und eine spezifische Aktivität von mindestens $7 \times 10^6$ Einheiten/mg aufweist.

3. Eine Interferonart nach Anspruch 1, die im wesentlichen die folgende Aminosäure-zusammensetzung aufweist:

## 0 117 470

| Aminosäure | Reste |
|---|---|
| Asx | 14.6±1.3 |
| Thr | 7.0±0.4 |
| Ser | 9.1±1.4 |
| Glx | 15.6±0.7 |
| Pro | 9.7±1.4 |
| Gly | 14.4±0.3 |
| Ala | 12.0±1.5 |
| Cys | 0.6±0.5 |
| Val | 7.4±0.7 |
| Met | 2.3±1.0 |
| Ile | 5.0±0.3 |
| Leu | 9.2±0.3 |
| Tyr | 2.9±0.4 |
| Phe | 4.9±1.0 |
| His | 3.5±0.7 |
| Lys | 20.3±0.4 |
| Arg | 5.7±0.4 |

4. Eine Interferonart nach Anspruch 2, die im wesentlichen die folgende Aminosäurezusammensetzung aufweist:

| Aminosäure | Reste |
|---|---|
| Asx | 16.5±0.4 |
| Thr | 7.4±0.6 |
| Ser | 10.8±1.0 |
| Glx | 16.4±1.4 |
| Pro | 8.0±0.4 |
| Gly | 14.3±1.9 |
| Ala | 11.3±0.7 |
| Cys | 0.6±0.4 |
| Val | 8.9±0.9 |
| Met | 0.8±0.8 |
| Ile | 6.0±0.3 |
| Leu | 12.9±0.1 |
| Tyr | 2.1±0.1 |
| Phe | 5.2±0.8 |
| His | 2.2±0.3 |
| Lys | 13.6±3.1 |
| Arg | 8.4±1.0 |

5. Eine Interferonart nach Anspruch 1 die bei 0,26M NaCl (pH 7) bei einer Durchflußrate von 0,1 bis 1,0 ml/min, einer Temperatur von −10°C bis 30°C und einem Druck von 0,7 bis 70 bar aus einer HPCL-Kationenaustausch-50×50 mm-Säule eluiert, nach Äquilibrierung in 10 mM Natriumphosphat (pH 7), 20% Vol/Vol Ethylenglycol.

6. Eine Interferonart nach Anspruch 2, die bei 0,25 M NaCl (pH 7) bei einer Durchflußrate von 0,1 bis 1,0 ml/min, einer Temperatur von −10°C bis 30°C und einem Druck von 0,7 bis 70 bar aus einer HPCL-Kationenaustausch-50×50 mm-Säule eluiert, nach Äquilibrierung in 10 mM Natriumphosphat (pH 7), 20% Vol/Vol Ethylenglycol.

7. Ein pharmazeutisches Präparat, das für parenterale Anwendung bei der Behandlung viraler und neoplastischer Krankheitsstadien geeignet ist, das eine kleine, wirksame Menge der Interferonart nach Anspruch 1 und eine große Menge eines konventionellen, pharmazeutischen, parenteral anwendbaren Trägermaterials enthält.

8. Ein pharmazeutisches Präparat, das für die parenterale Anwendung bei der Behandlung viraler und neoplastischer Krankheitsstadien geeignet ist, das eine kleine, wirksame Menge der Interferonart nach Anspruch 2 und eine große Menge eines konventionellen, pharmazeutischen, parenteral anwendbaren Trägermaterials enthält.

9. Eine Zusammensetzung, die eine Mischung aus (a) einer menschlichen Interferon-gamma-Art des Subtyps 21K gemäß Anspruch 1, in im wesentlichen homogener, biologisch aktiver Form und (b) eine menschliche Interferon-gamma-Art des Subtyps 26K gemäß Anspruch 2 in im wesentlichen homogener und biologisch aktiver Form enthält.

10. Ein pharmazeutisches Präparat, das für die parenteral Anwendung bei der Behandlung viraler und neoplastischer Krankheitsstadien geeignet ist, daß eine kleine wirksame Menge einer Zusammensetzung

8

nach Anspruch 9 und eine große Menge eines konventionellen, pharmazeutischen, parenteral anwendbaren Trägermaterials enthält.

11. Verfahren zum Herstellen von menschlichen Immun-Interferonen (IFN-γ), Subtypen 21K gemäß Anspruch 1, und 26K gemäß Anspruch 2 als im wesentlichen homogene und biologisch aktive Proteine in dem die folgenden Verfahrensstufen in Kombination angewendet werden:

a. Mischen einer wässrigen Lösung des Interferons-gamma in ungereinigtem Zustand mit einem Glasmaterial bestimmter Porengröße, so daß das genannte Interferon an das genannte Adsorbens adsorbiert sowie darauffolgendes Eluieren des genannten Interferons von dem genannten Adsorbens und Erhalten des genannten Interferons in ausgewählten Fraktionen des genannten Eluats in einem Zustand erhöhter Reinheit;

b. Konzentrieren der ausgewählten genannten Fraktionen, die im Verfahrensschritt(a) erhalten wurden durch Ultrafiltration an einer Membran und Erhalten des genannten Interferons in der durch die genannte Membran zurückgehaltenen Fraktion in einem Stadium höherer Konzentration und erhöhter Reinheit;

c Durchleiten der genannten, im Verfahrensschritt (b) erhaltenen Fraktion durch eine Kationenaustauschersäule, wobei die genannte Säule mit einem geeigneten Puffer äquilibriert wird und darauffolgendes Eluieren des genannten Interferons aus der genannten Säule mit einem ansteigenden Gradienten der Salzkonzentration oder alternativ dazu ansteigendem pH und unter mäßigem Druck und Erhalten der Subtypen 21K und 26K des Interferons-gamms als verschiedene distinkte Peaks in ausgewählten Fraktionen des genannten Eluats in einem Zustand homogener und biologisch aktiver Proteine.

12. Verfahren nach Anspruch 11, wobei Verfahrensschritt (c) unter HPLC-Bedingungen durchgeführt wird.

13. Verfahren nach Anspruch 11, wobei die Kationenaustauschersäule ein organisches Polymer enthält, das Sulfoalkylgruppen trägt.

14. Verfahren nach Anspruch 11, wobei das Interferon in Verfahrensstufe (c) durch ansteigende Salzkonzentration eluiert wird.

15. Verfahren nach Anspruch 11, wobei das Interferon in Verfahrensstufe (c) durch ansteigenden pH eluiert wird.

16. Verfahren nach Anspruch 11, wobei das Interferon in Verfahrensstufe (c) in Anwesenheit von Ethylenglycol eluiert wird.

17. Verfahren nach einem der Ansprüche 11 bis 16, wobei die genannte Säule eine Kationenaustauschersäule ist, die ein organisches Polymer enthält, das Sulfoalkylgruppen trägt, der genannte Puffer 10 mM-Natriumphosphat, pH 7,0, enthaltend 20% Vol/Vol Ethylenglycol, ist und der genannte Gradient 60 min einer linear von 0 bis 400 mM ansteigenden NaCl-Konzentration ist.

18. Verfahren nach einem der Ansprüche 11 bis 17, wobei in mindestens einer Fraktion des Verfahrensschrittes (c) der Subtyp 21K im wesentlichen frei vom Subtyp 26K eluiert wird und in mindestens einer anderen Fraktion des Verfahrensschritts (c) der Subtyp 26K im wesentlichen frei vom Subtyp 21K eluiert wird, wobei die Subtypen 21K und 26K in im wesentlichen homogener Form gleichzeitig hergestellt werden.

19. Verfahren nach Anspruch 11, wobei die Membran im Verfahrensschritt (b) ein Ausschlußmolekulargewicht von etwa 10,000 Datons aufweist.

**Revendications**

1. Espèce d'interféron gamma humain, de sous-type 21K, sous forme pratiquement homogène et biologiquement active, qui présente une masse moléculaire d'approximativement 21 000 et une activité spécifique d'au moins environ $7 \times 10^6$ unités/mg.

2. Espèce d'interféron gamma humain, de sous-type 26K, sous forme essentiellement homogène et biologiquement active, présentant une masse moléculaire d'approximativement 26 000 et une activité spécifique d'au moins environ $7 \times 10^6$ unités/mg.

3. L'espèce d'interféron de la revendication 1, ayant essentiellement la composition en amino-acides suivante:

# 0 117 470

| Amino-acides | Résidus |
|---|---|
| Asx | 14.6±1.3 |
| Thr | 7.0±0.4 |
| Ser | 9.1±1.4 |
| Glx | 15.6±0.7 |
| Pro | 9.7±1.4 |
| Gly | 14.4±0.3 |
| Ala | 12.0±1.5 |
| Cys | 0.6±0.5 |
| Val | 7.4±0.7 |
| Met | 2.3±1.0 |
| Ile | 5.0±0.3 |
| Leu | 9.2±0.3 |
| Tyr | 2.9±0.4 |
| Phe | 4.9±1.0 |
| His | 3.5±0.7 |
| Lys | 20.3±0.4 |
| Arg | 5.7±0.4 |

4. L'espèce d'interféron de la revendication 2, ayant essentiellement la composition en amino-acides suivante:

| Amino-acides | Résidus |
|---|---|
| Asx | 16.5±0.4 |
| Thr | 7.4±0.6 |
| Ser | 10.8±1.0 |
| Glx | 16.4±1.4 |
| Pro | 8.0±0.4 |
| Gly | 14.3±1.9 |
| Ala | 11.3±0.7 |
| Cys | 0.6±0.4 |
| Val | 8.9±0.9 |
| Met | 0.8±0.3 |
| Ile | 6.0±0.3 |
| Leu | 12.9±0.1 |
| Tyr | 2.1±0.1 |
| Phe | 5.2±0.8 |
| His | 2.2±0.3 |
| Lys | 13.6±3.1 |
| Arg | 8.4±1.0 |

5. L'espèce d'interféron de la revendication 1, qui élue à NaCl 0,26 M (pH 7), à partir d'une colonne échangeuse de cation de 5×50 mm, de chromatographie liquide à haute performance (HPLC), à une vitesse d'écoulement de 0,1 à 1 ml par minute, à une température de −10° à 30°C, et une pression de 0,7 à 70 bars, après équilibrage dans de l'éthylène glycol à 20% en volume/volume de phosphate sodique 10 mM (pH 7).

6. L'espèce d'interféron de la revendication 2, qui élue à NaCl 0,25 M (pH 7), à partir d'une colonne échangeuse de cation de 5×50 mm, de chromatographie liquide à haute performance (HPLC), à une vitesse d'écoulement de 0,1 à 1 ml par minute, à une température située entre −10° et 30°C, et une pression de 0,7 à 70 bars, après équilibrage dans de l'éthylène glycol à 20% en volume/volume de phosphate sodique 10 mM (pH 7).

7. Préparation pharmaceutique convenant à l'administration parentérale pour le traitement de maladies virales et néoplasiques, qui comprend une quantité mineure efficace de l'espèce d'interféron de la revendication 1, et une quantité majeure d'un véhicule parentéral pharmaceutique, classique.

8. Préparation pharmaceutique convenant à l'administration parentérale pour le traitement de maladies virales et néoplasiques, qui comprend une quantité mineure, efficace, de l'espèce d'interféron de la revendication 2, et une quantité majeure d'un véhicule pharmaceutique parentéral classique.

9. Compositions comprenant un mélange de (a) une espèce d'interféron gamma humain, de sous-type 21K selon la revendication 1, sous forme essentiellement homogène et biologiquement active, et (b) une espèce d'interféron gamma humain, de sous-type 26K selon la revendication 2, sous forme essentiellement homogène et biologiquement active.

10. Préparation pharmaceutique convenant à l'administration parentérale pour le traitement des maladies virales et néoplasiques qui comprend une quantité mineure, efficace, de la composition selon la revendication 9, et une quantité majeure d'un véhicule pharmaceutique parentéral, classique.

11. Procédé pour la production d'interféron humain immun (IFN-γ) de sous-types 21K selon la

10

**0 117 470**

revendication 1, et 26K selon la revendication 2, sous forme de proteines pratiquement homogènes et biologiquement actives, qui comprend en combinaison:

(a) mélange d'une solution aqueuse de IFN-γ à l'état impur avec un produit en verre à pores réglés de manière à ce qu'il y ait adsorption de cet interféron sur cet adsorbant, suivi d'une élution de l'interféron à partir de l'adsorbant et obtention de fraction choisies d'interféron de cet éluat à l'état de pureté accrue;

(b) concentration des fraction choisies obtenues au stade (a) par ultrafiltration sur une membrane, et obtention de l'interféron, dans la fraction retenue par la membrane, à un état de concentration supérieure et de pureté améliorée;

c) passage de la fraction obtenue au stade (b) par une colonne échangeuse de cation, équilibrée à l'aide d'un tampon convenable, ensuite élution de cet interféron à partir de la colonne à l'aide d'un gradient de concentration saline accrue, ou bien d'une augmentation de pH, et sous pression modérée et obtention de sous-type 21K et 26K d'IFN-γ sous forme de plusieurs pics distincts dans des fractions choisies de cet éluat, à l'état de protéines homogènes et biologiquement actives.

12. Procédé selon la revendication 11 dans lequel on opère au stade (c) dans des conditions de chromatographie liquide à haute performance (HPLC).

13. Procédé selon la revendication 11, dans lequel la colonne échangeuse de cation comprend un polymère organique portant des groupement sulfoalkyle.

14. Procédé selon la revendication 11, dans lequel au stade (c), l'interféron est élué par concentration croissante de sel.

15. Procédé selon la revendication 11, dans lequel, au stade (c) l'interféron est élué par accroissement du pH.

16. Procédé selon la revendication 11, dans lequel, au stade (c), l'élution de l'interféron se déroule en présence d'éthylène glycol.

17. Procédé selon une des revendications 11 à 16, dans lequel la colonne est une colonne échangeuse de cation comprenant un polymère organique portant des groupements sulfoalkyle, le tampon est le phosphate sodique 10 mM pH 7 renfermant 20% en volume/volume de éthylène glycol et le gradient est de concentration de NaCl linéairement augmentée de 0 à 400 mM en 60 min.

18. Procédé selon une des revendications 11 à 17, dans lequel au moins dans une fraction de stade (c), le sous-type 21K est élué essentiellement exempt de sous-type 26K, et dans au moins une autre fraction de stade (c), le sous-type 26K et élué essentiellement exempt de sous-type 21K, de sorte que le sous-types 21K et 26K sont co-produits sous la forme essentiellement homogène.

19. Procédé selon la revendication 11, dans lequel la membrane au stade (b) a un pouvoir de séparation de poids moléculaire de l'ordre de 10 000 daltons.

11